# EUROPEAN PATENT APPLICATION

(11) **EP 3 932 866 A1**
(43) Date of publication of application: **05.01.2022**
(21) Application number: 20184045.1
(22) Date of filing: 03.07.2020
(51) Int. Cl.: C01F 17/30, B82Y 20/00, B82Y 30/00, B82Y 40/00

(54) **NANOPARTICLE WITH A BUFFER LAYER**

(71) Applicant: Universiteit van Amsterdam, 1012 WX Amsterdam (NL)
(72) Inventor: ZHANG, Hong, 1012 WX Amsterdam (NL); FENG, Yansong, 1012 WX Amsterdam (NL)
(74) Representative: V.O.

(57) **Abstract**

In accordance with the present invention there is provided a nanoparticle, comprising
- a core,
- an outer layer, and
- a buffer layer located between said core and said outer layer,
wherein the buffer layer prevents energy transfer between the core and the outer layer.

In another aspect of the invention there is provided a nanoparticle as described herein for use in therapy, a nanoparticle according as described herein for use in treatment of a tumor, and a nanoparticle as used herein for use in *in vivo* imaging.

According to another aspect of the invention there is provided a method for the production of a nanoparticle as described herein, comprising the steps of
- heating of a solution comprising core precursors, followed by
- injection of a dispersion comprising buffer layer precursors, followed by
- injection of a dispersion comprising outer layer precursors.

Another aspect of the invention is a method for *in vivo* imaging or treatment or both of a human or animal, comprising the steps of:
- administering nanoparticles as described herein to a patient,
- irradiating at least part of the patient's body with one or more types of radiation.

According to yet another aspect of the invention there is provided the use of a nanoparticle as described herein for therapy, *in vivo* imaging, or both.

## Description

### BACKGROUND OF THE INVENTION

The invention is in the field of radiation therapy. In particular the present invention is directed to a nanoparticle, a nanoparticle for use in therapy and *in vivo* imaging, and a method for the preparation of a nanoparticle.

Radiation therapy, also known as radiotherapy, is often used for treatment of cancer, tumors, or other malignancies. In radiotherapy, ionizing radiation is used to kill malignant cells or to slow their growth by damaging the DNA of these cells.

Another approach for killing or slowing the growth of malignant cells is photodynamic therapy (PDT). Typically, in photodynamic therapy, a photosensitizer is activated by electromagnetic radiation to produce cytotoxic moieties, that damage or kill cells that are exposed to these moieties. If compounds comprising such photosensitizers are activated in close proximity to malignant cells, this cytotoxicity can be used to kill malignant cells, such as cancer cells.

Whereas radiation therapy damages cells primarily by damaging the DNA, the cytotoxic species produced in photodynamic therapy can also damage other parts of cells, such as cell membranes. Therefore, radiotherapy and photodynamic therapy can be used supplementary to each other. Depending on which photosensitizer is used, activation of the photosensitizer can be done using the same radiation that is used for radiotherapy or using radiation of a different type or wavelength.

Traditionally, photosensitizers used in photodynamic therapy are activated by electromagnetic radiation with wavelengths in the ultraviolet or visible region. Such wavelengths are used for instance in EP 1100366. A disadvantage of using ultraviolet (UV) or visible (vis) light is the limited penetration depth of these types of radiation, which generally restricts the use of PDT with ultraviolet or visible radiation to superficial lesions (*e.g.* skin cancer).

In US20160271251, US20130158336, and Angew. Chem. Int. Ed. 2015, 54, 1770 -1774, materials comprising a luminescent compound and a photosensitizer are described. The photosensitizer can absorb light that is emitted by the luminescent compound when the luminescent compound is activated by high-energy radiation. Using such materials, a deeper penetration depth can be achieved for PDT. However, a downside of this radiative energy transfer from luminescent compounds towards the photosensitizers is not very efficient.

Another problem related to radiotherapy and photodynamic therapy is that healthy cells close to the malignant cells are also exposed to the harmful radiation and/or cytotoxicity. In order to make sure that all malignant cells are treated during radiotherapy, it is common that a slightly region that is slightly larger than the malignancy is irradiated. If this region is too large, this can result in a large amount of healthy cells getting damaged, which can have serious negative effects on a patient. Therefore, it is desirable order to minimize the region that needs to be irradiated, and to expose as little healthy cells as possible to damaging radiation. One of the ways in which this can be achieved is to combine radiotherapy with imaging techniques. If the location of malignant cells can be determined very accurately, it means that the safe margin around the malignancy that needs to be irradiated can be made smaller, resulting in less exposure of healthy cells to damaging radiation.

To this end, it can be beneficial to use particle that can act both as an agent for therapy (such as photodynamic therapy) as well as for imaging. In US20160271251, particles that are used for photodynamic therapy comprising a magnetic resonance imaging (MRI) contrast agent are described. In J. Am. Chem. Soc. 2013, 135, 6494-6503, particles with an upconversion nanoparticle core and a porous silica shell are disclosed. The particle can deliver a chemotherapy drug to tumors, and the upconversion nanoparticle core may be used for imaging.

However, a problem associated with using the same particle for both imaging and therapy is that the two functions can negatively influence each other. For instance, if a particle is irradiated with electromagnetic radiation for the purpose of imaging, some of the energy that is absorbed in the imaging process that is supposed to converted into electromagnetic radiation of another wavelength may also be transferred to the parts of the particle that are responsible for the therapeutic functionality of the particle. Such energy transfer processes may lead to several problems. For example, imaging may become less accurate or less efficient, because this unintended energy transfer to other parts of the particle results in less of the absorbed energy being converted for the purpose of imaging. Another negative effect may be that when the particles are used for imaging in order to accurately determine which region in the tissue should be treated, energy transfer towards a region of the particle that is used for treatment may result in activation of therapy, such as damaging of cells by cytotoxicity, in unwanted locations.

An object of the present invention is to address one or more of these problems associated with radiation therapy. Specifically, an object of the present invention is to provide a possibility to effectively combine radiation therapy, photodynamic therapy, and/or imaging while limiting unwanted interference between these applications.

### BRIEF SUMMARY OF THE INVENTION

In accordance with the present invention there is provided a nanoparticle, comprising
- a core,
- an outer layer, and
- a buffer layer located between said core and said outer layer,
wherein the buffer layer prevents energy transfer between the core and the outer layer.

The buffer layer prevents energy transfer from the core to the outer layer, and/or from the outer layer to the core. By preventing these energy transfer processes, the core and/or outer layer of the particle can be used efficiently for applications such as medical imaging and treatment.

In another aspect of the invention there is provided a nanoparticle as described herein for use in therapy, a nanoparticle according as described herein for use in treatment of a tumor, and a nanoparticle as used herein for use in *in vivo* imaging.

According to another aspect of the invention there is provided a method for the production of a nanoparticle as described herein, comprising the steps of
- heating of a solution comprising core precursors, followed by
- injection of a dispersion comprising buffer layer precursors, followed by
- injection of a dispersion comprising outer layer precursors.

Another aspect of the invention is a method for *in vivo* imaging or treatment or both of a human or animal, comprising the steps of:
- administering nanoparticles as described herein to a patient,
- irradiating at least part of the patient's body with one or more types of radiation.

According to yet another aspect of the invention there is provided the use of a nanoparticle as described herein for therapy, *in vivo* imaging, or both.

### BRIEF DESCRIPTION OF THE DRAWINGS

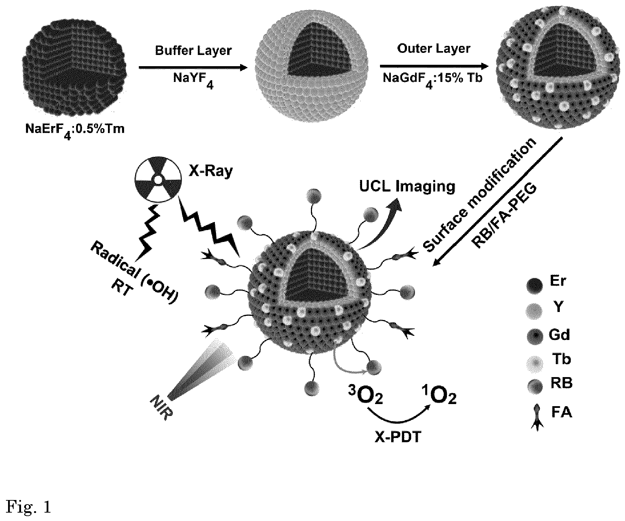

Figure 1 is a schematic overview of the design and synthesis, as well as the mechanisms of upconversion imaging and combined radiotherapy and X-ray mediated photodynamic therapy of nanoparticles according to an embodiment of the invention.
Figure 2 shows electron microscopy images of nanoparticles according to an embodiment: (a) TEM image of the core NaErF₄: 0.5% Tm; (b) TEM images and (c) HRTEM image of NPs; (d) HRTEM image of an individual NPs; (e) HAADF-STEM image of NPs (left-up) and the STEM-EDS element mapping (Er, Y, Tb, Gd, Na and F) images of NP
Figure 3 shows (a) UCL spectra of NPs under laser excitation at 808 nm, 980 nm and 1530 nm, respectively; (b) emission spectrum of a nanoparticle under X-ray irradiation; (c) schematic diagram of energy transfer interactions in a nanoparticle according to an embodiment of the invention, when subjected to NIR or X-ray irradiation; (d) buffer layer thickness dependent upconversion luminescence of nanoparticles according to an embodiment of the invention under 980 nm laser excitation (The buffer layer NaYF₄ was 0, 1, 2, 3 or 4 equivalent (e.q.) of the core).
Figure 4 shows (a) diameter, (b) zeta potential, (c) FTIR spectra of nanoparticles according to embodiments of the invention, (d) luminescence spectra of nanoparticles under 254 nm excitation, (e) dynamic curves of emission by nanoparticles and rose bengal (RB) upon excitation with 378 nm, (f) relative diphenylisobenzofuran (DPBF) consumption by nanoparticles using different irradiation wavelengths.
Figure 5 shows (a) confocal images of MCF-7 cells incubated with 50 µg/mL, 100 µg/mL and 200 µg/mL nanoparticles according to an embodiment of the invention, where the nuclei were stained with DAPI. The UCL images were taken with 650 nm UCL under 980 nm laser irradiation. RB images were taken with 590 nm emission under 540 nm laser irradiation; (b) different aisle confocal images of MCF-7 cells incubated with nanoparticles according to an embodiment of the invention. UCL-Red, UCL-Green and UCL-RB images were taken under 980 nm irradiation with 650 nm, 540 nm and 590 nm UCL, respectively.
Figure 6 shows (a) viability of cells incubated with different concentration UIRPPs plus NIR laser (808 nm, 980 nm or 1530 nm irradiation. (b) cell viability of different groups, including the one incubated with saline, with different concentration RB, with NPs or UIRPPs plus X-ray irradiation; (c) apoptosis of MCF-7 cell groups, including the one incubated with saline, UIRPPs, UIRPPS + 980 nm irradiation, under 980 nm laser irradiation only, NPs + X-ray, different concentration UIRPPs (50, 100 or 200 µg/mL) + X-ray. The MCF-7 cells were stained by Annexin V-FITC and analyzed by FAC Scan.
Figure 7 shows an XRD pattern of the core NaErF₄:0.5%Tm and multi-layer Er@Y@Gd/Tb NPs, the standard diffraction pattern of hexagonal phase NaYF₄ is for reference. (JCPDS No. 16-0334).
Figure 8 shows luminescence spectra of Er@Y@Gd/Tb NPs under UV light (254 nm) or NIR (980 nm) excitation and the absorption spectrum of RB.
Figure 9 shows the UCL spectra of Er@Y@Gd/Tb NPs upon (a) 808 nm or (b) 1530 nm laser excitation with buffer layer of different thicknesses (the buffer layer NaYF₄ was 0, 1, 2, 3 or 4 e.q. of the core).
Figure 10 shows a ten days track of UCL spectra of UIRPPs after preparation.
Figure 11 shows UV-Vis absorption spectra of the solutions containing DPBF incubated with (a) 0.1 mg/mL RB and (b-d) 2 mg/mL UIRPPs. (b-d) were under irradiation of the laser at 808 nm, 980 nm or 1530 nm, respectively. The irradiation time duration was marked in the figures.
Figure 12 shows UV-Vis absorption spectra of solutions containing DPBF incubated with (a) 1.5 mg/mL UIRPPs, (b) 2.5 mg/mL UIRPPs and (c) 5 mg/mL UIRPPs subject to X-ray irradiation with time duration marked in the figures.
Figure 13 shows the cell viability of MCF-7 incubated with different concentration UIRPPs (50, 100, 200 or 400 µg/mL), and the cell viability subject to the NIR laser irradiation (808 nm, 980 nm or 1530 nm).
Figure 14 shows the confocal images of MCF-7 cells incubated with 100 µg/mL UIRPPs, the nuclei were stained with DAPI; the UCL images with irradiation of 808 nm or 980 nm and emission of 650 nm (red) or green (540 nm), respectively.
Figure 15 shows Z-scan confocal images of MCF-7 cells incubated with 100 µg/mL UIRPPs, the nuclei were stained with DAPI; the UCL images under irradiation of 980 nm laser and 650 nm emission; the RB images under irradiation of 540 nm and 590 nm emission.

### DETAILED DESCRIPTION OF THE INVENTION

According to the invention there is provided a nanoparticle, comprising
- a core,
- an outer layer, and
- a buffer layer located between said core and said outer layer,
wherein the buffer layer prevents energy transfer between the core and the outer layer.

The buffer layer prevents energy transfer between the core and the outer layer by preventing energy transfer from the core to the outer layer, and/or from the outer layer to the core. By preventing these energy transfer processes, the core and/or outer layer of the particle can be used efficiently for applications such as medical imaging and treatment.

Energy transfer, as used herein, refers to electronic energy transfer, *i.e*., the radiative or non-radiative transfer of energy from an electronically excited donor to an acceptor. As used herein, energy transfer does not refer to other types of processes in which energy is transferred from one system to another, such as mechanical energy transfer or vibrational energy transfer.

Radiation therapy (RT), or radiotherapy, refers to treatment of cancer, tumor or other malignancies using ionizing radiation.

Radiosensitizer, as used herein, refers to any compound that, when used in combination with ionizing radiation (*i.e*., particle radiation such as proton radiation, or electromagnetic radiation with photon energy greater than 10 eV such as X-rays or y-rays), enhances the damage to cells caused by the ionizing radiation.

Photosensitizer, as used herein, refers to any compound that, when used in combination with non-ionizing radiation (*i.e*., electromagnetic radiation with photon energy of 10 eV or lower, including ultraviolet, visible, near-infrared, and infrared radiation), enhances the damage to cells caused by the non-ionizing electromagnetic radiation.

In some cases, a certain compound can act both as a photosensitizer and as a radiosensitizer, depending on the conditions. An example of such a compound is rose bengal (RB). RB can act as a photosensitizer when irradiated with green light. However, RB can also act as a radiosensitizer under influence of X-ray irradiation, either activated directly by X-rays or indirectly through energy transfer from another compound that is activated by X-rays.

A specific type of therapy in which photosensitizers and/or radiosensitizers are applied is photodynamic therapy (PDT). In this therapy, photo- and/or radiosensitizers are activated by radiation, which causes them to produce oxygen radicals and/or singlet oxygen species, which cause cytotoxicity in the direct vicinity of the photo-and/or radiosensitizers. Generally, photodynamic therapy refers to the therapy in which photosensitizers provide the cytotoxic effects when activated by non-ionizing electromagnetic radiation. However, photodynamic therapy can also encompass therapy in which radiosensitizers provide the cytotoxic effects performed when activated by ionizing radiation. In the latter case, the therapy is sometimes also referred to as X-ray induced or X-ray mediated photodynamic therapy, or X-PDT.

In case the nanoparticle has a therapeutic function, it is preferred that the therapeutic functionality is provided by the outer layer, since therapeutic effects such as cytotoxicity typically occur only very locally.

The core, the outer layer, and the buffer layer may have a wide variety of compositions. These compositions can be chosen depending on the desired functionality of the core and the outer layer. These functionalities include imaging techniques such as magnetic resonance imaging (MRI), computerized tomography (CT), fluorescence imaging techniques, and upconversion luminescence (UCL) imaging, as well as therapeutic functionalities, such as functionality in photodynamic therapy, drug delivery, targeting of tumor cells. Each of these functionalities require different compositions of the core and/or outer layer. Therefore, the composition of the buffer layer can be chosen based on the functionality of the core and/or the outer layer and the composition that is connected with this functionality.

In order to choose a buffer layer that is effective in preventing energy transfer from an excited state in the core to the outer layer and/or from an excited state in the outer layer to the core, a buffer layer composition may be chosen of which the energy levels do not match the conditions for any radiative or nonradiative energy transfer between the buffer layer and the core and/or between the buffer layer and the outer layer. In this way, energy transfer from the core to the outer layer and/or from the outer layer to the core cannot proceed *via* the buffer layer.

In order to also prevent direct energy transfer from the core to the outer layer and/or from the outer layer to the core (*i.e*., energy transfer in which the buffer layer is not involved), the thickness of the buffer layer should be high enough, because energy transfer processes are heavily dependent on the distance between the materials between which the energy is transferred. The preferred thickness of the buffer layer may depend on the specific materials of the core, the buffer layer, and the outer layer. Preferably, the thickness of the buffer layer is 1 nm or more, more preferably 2 nm or more, even more preferably 3 nm or more. The thickness of the buffer layer may be as high as 30 nm, but is preferably lower, such as 5-20 nm or 3-10 nm. For example, it was shown by the present inventors that a buffer layer of NaYF₄ with a thickness of 2.6 nm or more effectively prevented energy transfer between a core comprising NaYF₄ doped with rare-earth ions and an outer layer comprising NaGdF₄ doped with rare-earth ions.

Another way to determine whether a certain combination of compositions of core, buffer layer and outer layer can be used for a nanoparticle according to the invention is to synthesize a particle comprising a core, a buffer layer and an outer layer, and test whether excitation of the outer layer by radiation leads to interference with the function of the core, and/or vice versa. For instance, if the core of the nanoparticle has a composition making it suitable for upconversion luminescence imaging using near-infrared (NIR) radiation, and the outer layer has a composition making it suitable for X-PDT, the buffer layer should prevent interference with the imaging functionality of the core in case the outer layer is excited by X-rays, and the X-PDT functionality should not be activated by excitation of the core with NIR radiation. This can for instance be assessed by measuring cytotoxicity of the nanoparticle upon irradiation with NIR radiation, or by testing and comparing imaging accuracy and efficiency with and without X-ray irradiation.

It is preferred that the core, the outer layer, and the buffer layer are crystalline, because this generally results in higher efficiency of the functionality of the core and the outer layer, such as radiation-induced imaging and therapy. Another advantage is that the separation between the core and the different layers of the nanoparticles is more pronounced in case the core, the buffer layer and the outer layer are crystalline.

Preferably, the core, the buffer layer and the outer layer have substantially the same crystal structure, such as a hexagonal crystal structure. If the core, the buffer layer and the outer layer have substantially the same crystal structure, less defects will be present at the interface between the different layers. This results in the nanoparticles functioning more efficiently. Other advantages of the core, the buffer layer and the outer layer having substantially the same crystal structure are that this may result in the particles having larger mechanical strength. Also, it may have positive influence on the synthesis of the particles, especially in case the synthesis of the nanoparticles includes crystal growth.

If the core, the buffer layer and the outer layer have substantially the same crystal structure, the lattice mismatch between the buffer layer and the core and between the buffer layer and the outer layer is sufficiently small to accommodate layer growth. If the lattice mismatch is too large, this can result in the increase of defects between the layers, and it can have a negative effect on the crystallinity of the layers. Preferably, the lattice mismatch is 25% or less, more preferably 10% or less, such as 5% or less.

In a preferred embodiment, the core of the nanoparticle as described herein comprises one or more rare-earth elements. This enables the use of nanoparticles of the invention for imaging. Rare-earth containing nanoparticles, for example upconversion nanoparticles, can advantageously be used for imaging in deeper layers of tissue by using near-infrared radiation to excite the material, thereby causing emission in the near-infrared and/or deep red spectral region, without the adverse effects of imaging techniques such as computerized tomography (CT) using X-rays. In a preferred embodiment, the core of the nanoparticle as described herein comprises an upconversion material.

Preferably, the core, the buffer layer, and/or the outer layer comprise an oxide or fluoride material comprising one or more rare-earth elements. Examples of such materials include CaO-X₂O₃ wherein X is a rare-earth element. Other examples include NaXF₄, wherein X is a rare-earth element, optionally doped with one or more other rare-earth elements. The presence of rare-earth elements in these materials may enable the use of these materials for imaging and/or as a radiosensitizer.

In case the nanoparticle is used for imaging, the choice of rare-earth elements influences the wavelength at which luminescence occurs. For instance, when irradiated with NIR radiation with a wavelength of 980 nm, NaYF₄ doped with Er³⁺ and Yb³⁺ emits light mainly at 650nm and 1530 nm, whereas NaYF₄ doped with Yb³⁺ and Tm³⁺ emits mainly light of 808 nm. This results in a nanoparticle which can be used for imaging using NIR irradiation, for instance through upconversion luminescence (UCL). In addition to the examples above, other lanthanide or rare-earth elements or combination thereof, present in any amount in which they cause emission in the red, deep red, and/or NIR spectral range upon NIR irradiation, can also be used to provide the nanoparticle, preferably the core of the nanoparticle, with imaging functionality.

Preferably, the outer layer of the nanoparticle according to the invention can act as a radiosensitizer, and/or act as a substrate for X-ray induced photodynamic therapy. In case the outer layer is used for X-ray induced photodynamic therapy, rare earth elements in the outer layer can be chosen such that the energy level scheme of the outer layer facilitates an efficient energy transfer to sensitizers that may be anchored on the particle surface. For instance, the outer layer may comprise NaGdF₄ doped with Tb³⁺ or NaGdF₄ doped with Tb³⁺ and Ce³⁺. In addition, rare earth elements which have emission in UV to visible spectral region under X-ray radiation can be used as radiosensitizing dopant in the outer layer.

For rare-earth doped NaXF₄ compounds, the amount of dopant atoms can vary to a large extent and can be chosen and optimized for optimal imaging and/or therapeutic effects. For example, the one or more dopants may be present in an amount of 0-50 atom%, Preferably, the dopants are present in an amount of 0.1-30 atom%, such as 0.5-20 atom%.

According to an embodiment of the invention, the nanoparticle as described herein further comprises one or more photosensitizers, radiosensitizers, and/or tumor targeting moieties attached to the outer layer. The photosensitizers and/or radiosensitizers may be activated directly by non-ionizing and/or ionizing radiation, or they can be activated indirectly *via* radiosensitizing ions in the outer layer that are excited by non-ionizing and/or ionizing radiation. This indirect activation of the photosensitizers and/or radiosensitizers can be caused by non-radiative and/or radiative energy transfer from the excited atoms in the outer layer. Preferably, indirect activation of the photosensitizers and/or radiosensitizers takes places by non-radiative energy transfer, such as via Forster resonance energy transfer (FRET), because very high efficiencies can be achieved with FRET.

Preferably, the photosensitizers, radiosensitizers, and/or tumor targeting moieties are anchored to the outer layer via a functionalized groups, such as poly(allylamine) groups.

A wide variety of photosensitizers, radiosensitizers, and/or tumor targeting moieties can be anchored to the outer layer of the nanoparticle. They can be chosen according to the desired function of the nanoparticles. Photo- and/or radiosensitizer that can be used include tirapazamine (TPZ); rose bengal (RB); porphyrin derivatives such as porfimer sodium, hematoporphyrin (HP), protoporphyrin IX (PpIX); phthalocyanine (Pc) derivatives, such as ZnPc, AlC₄PC; chlorin derivatives such as chlorin e6 (Ce6), meta-tetra(hydroxyphenyl)chlorin (mTHPC); benzoporphyrin derivatives such as verteporfin; NIR-absorbing photosensitizers such as indocyanine green (ICG), cypate, naphthalocyanines; as well as other compounds that can be activated by ultraviolet or visible light. Tumor targeting moieties that can be used include monoclonal antibodies, polyunsaturated fatty acids, folic acid (FA), hyaluronic acid, and oligopeptides.

Another aspect of the invention is the nanoparticle as described herein for use in therapy. The core and/or outer layer of the nanoparticle as described herein may have provide the nanoparticle with therapeutic functionality, such as transportation and delivery of drugs, and cytotoxicity.

Another aspect of the invention is the nanoparticle as described herein for use in treatment of a tumor. In case the nanoparticle exhibits cytotoxicity, it can be used for instance for killing tumor cells or slowing down the growth of tumors. Alternatively or additionally, the nanoparticles can transport drugs that are effective in the treatment of malignancies, and deliver these drugs specifically in the proximity of malignant cells.

Another aspect of the invention is the nanoparticle as described herein for use in *in vivo* imaging. Preferably, in case the core and/or outer layer of the nanoparticle comprise an nanoparticle that can be used for imaging in deep red or NIR spectral region using *e.g*. NIR radiation, the nanoparticles can be used for imaging in deep tissue, for instance imaging cancer, tumors, or other malignancies without exposing healthy cells to ionizing radiation. Advantageously, when nanoparticles according to the invention are used in for imaging as well as treatment of tumors, the imaging function can enable more targeted treatment of tumors using ionizing radiation, thereby limiting the amount of harmful radiation that healthy cells are exposed to.

Another aspect of the invention is a method for the production of a nanoparticle as described herein, comprising the steps of
- heating of a solution comprising core precursors, followed by
- injection of a dispersion comprising buffer layer precursors, followed by
- injection of a dispersion comprising outer layer precursors.

Preferably, in order to dissolve the core precursors, buffer layer precursors, and outer layer precursors, the solution is heated to a temperature of 200 °C or higher, preferably 250 °C or higher, such as 300 °C or higher.

The crystal growth preferably takes place in the absence of water and OH⁻. OH-, which can be contained in the reactants (such as rare earth chloride hexahydrate and NaOH, which are typically used in the synthesis of nanoparticles comprising rare earth elements) or solvents (such as H₂O) can readily be incorporated into the crystal lattice by substituting F- in the NaYF₄ sublattices, due to their similarities in terms electric charge, ion size and electronegativity. This is unwanted, because the incorporation of OH⁻ in the lattice may result in serious quenching of the luminescence during imaging.

In embodiments, the solution and dispersions contain less than 200 ppm of water by total weight of the solution or dispersion, preferably less than 100 ppm. More preferably, the solution and dispersions are substantially free of water.

In order to attach functional groups to the outer layer of a nanoparticle according to the invention, the method may further comprise the step of functionalization of the surface of the outer layer. Surface functionalization can be performed using ligand exchange. Preferred molecules for functionalization of the surface of the outer layer are poly(allylamine), as well as PEG derivatives such as poly(acrylamide) (PAAm) or poly(acrylic acid) (PAA).

After functionalization of the surface of the outer layer, functional groups, such as photosensitizers, radiosensitizers, tumor targeting moieties, and/or chemotherapy medicines can be bound to the to the functionalized nanoparticle. The functional groups may be bound to the nanoparticle using covalent bonds, and/or physical adsorption. Preferably, these functional groups are covalently bound to the functionalized outer layer, because this results in a more stable bond between the functional groups and the nanoparticle.

Another aspect of the invention is a method for *in vivo* imaging or treatment or both of a human or animal, comprising the steps of:
- administering nanoparticles as described herein to a patient,
- irradiating at least part of the patient's body with one or more types of radiation.

According to yet another aspect of the invention there is provided the use of a nanoparticle as described herein for therapy, *in vivo* imaging, or both.

### EXAMPLES

### Reagents

Y(CH₃COO)₃ *·x*H₂O, Tb(CH₃COO)₃ ·xH₂O, Er(CH₃COO)₃ *·x*H₂O, Tm(CH₃COO)₃ *·x*H₂O, Gd(CH₃COO)₃ *·x*H₂O, oleic acid (90%), 1-octadecene (ODE, 90%), NaOH, NH₄F, sodium oleate (NaOA), acetic acid, acetic anhydride, rose bengal (RB), poly(allylamine) 20 wt.% solution in water (PAAm), 6-bromohexanoic acid, N,N-dimethylformamide (DMF), N-(3-dimethylaminopropyl)-N'-ethylcarbodiimide hydrochloride (EDC-HCl), N-hydroxy-succinimide (NHS), 1,3-diphenylisobenzofuran (DPBF), folic acid (FA), N-hydroxysuccinimide functionalized polyethylene glycol (PEG-NHS), ethanol, acetone and cyclohexane were purchased from Sigma-Aldrich. All the chemicals were of analytical grade and were used without further purification.

### Example 1 - Synthesis of dry core β-NaErF₄:0.5%Tm nanoparticles

10 mL 1-octadecene and 10 mL oleic acid were heated up to 100 °C and kept under vacuum for 60 min. 0.995 mmol Er(CH₃COO)₃ *·x*H₂O and 0.005 mmol Tm(CH₃COO)₃ *·x*H₂O were then added in under nitrogen flow. Subsequently, 0.5 mL acetic anhydride was injected in the solution under nitrogen and the solution was heated up to 100 °C for 60 min. Acetic anhydride was removed under vacuum at 100 °C. Thereafter, 2.8 mmol sodium oleate was added under nitrogen flow and the atmosphere was switched to vacuum. After all reagents fully dissolved, 5.8 mmol NH₄F was added under nitrogen flow and the solution was heated at 100 °C under vacuum for 60 min to completely remove the water or other H₂O/OH-sources before forming the nanoparticles. Finally, the solution was heated up to 300 °C with a heating rate of 10 °C/min and reacted for another 60 min under dry nitrogen. After cooling down to room temperature, the core nanoparticles were obtained through centrifugation, washed with acetone and ethanol (twice) and finally dispersed in cyclohexane.

### Example 2 - Synthesis of α-NaYF₄ precursor

160 mL oleic acid and 160 mL ODE in 500 ml three necks flask were heated up to 100 °C and kept under vacuum for 60 min. 20 mmol Y(CH₃COO)₃ *·x*H₂O was then added in under nitrogen flow. Subsequently, 5 mL acetic anhydride was injected in the solution under nitrogen and heated up to 100 °C for 60 min. Acetic anhydride was removed under vacuum at 100 °C. Thereafter, 30 mmol NaOA was added under nitrogen flow and the atmosphere was switched to vacuum. After all reagents fully dissolved, 80 mmol NH₄F was added in under nitrogen flow and the solution was heated up at 100 °C under vacuum for 60 min to completely remove the water or other H₂O/OH⁻ sources before forming the nanoparticles. Finally, the solution was heated up to 200 °C with a rate of 10 °C/min and reacted for another 60 min under dry nitrogen. After cooling down to room temperature, the α-NaYF₄ precursor was obtained through centrifugation, and finally dispersed in 40 mL dry ODE.

### Example 3 - Synthesis of α-NaGdF₄:15%Tb precursor

The synthesis route for α-NaGdF₄:Tb was the same as that of α-NaYF₄, except that 17 mmol Gd(CH₃COO)₃ *·x*H₂O and 3 mmol Tb(CH₃COO)₃ *·x*H₂O were used.

### Example 4 - Synthesis of β-NaErF₄:Tm@NaYF₄@NaGdF₄:15%Tb (1:x:3, x = 0, 1, 2, 3 or 4) multi-layer nanoparticles (Er@Y@Gd/Tb NPs)

0.5 mmol core β-NaErF₄:Tm particles in 10 mL 1-octadecene and 10 mL oleic acid were heated up to 100 °C and kept under vacuum for 60 min. The solution was then heated up to 300 °C under nitrogen, followed by injection of 0.5x mmol (*x* = 0, 1, 2, 3 or 4) α-NaYF₄ in dry ODE (each time 0.5 mmol at 15 min interval) and then kept in 300 °C for 45 min. Afterwards, 1.5 mmol α-NaGdF₄:15%Tb was injected in three times (each time 0.5 mmol at 15 min interval) and reacted for other 45 min. After cooling down to room temperature, Er@Y@Gd/Tb nanoparticles (1:x:3) were obtained by centrifugation, washed with acetone and ethanol and finally dispersed in cyclohexane.

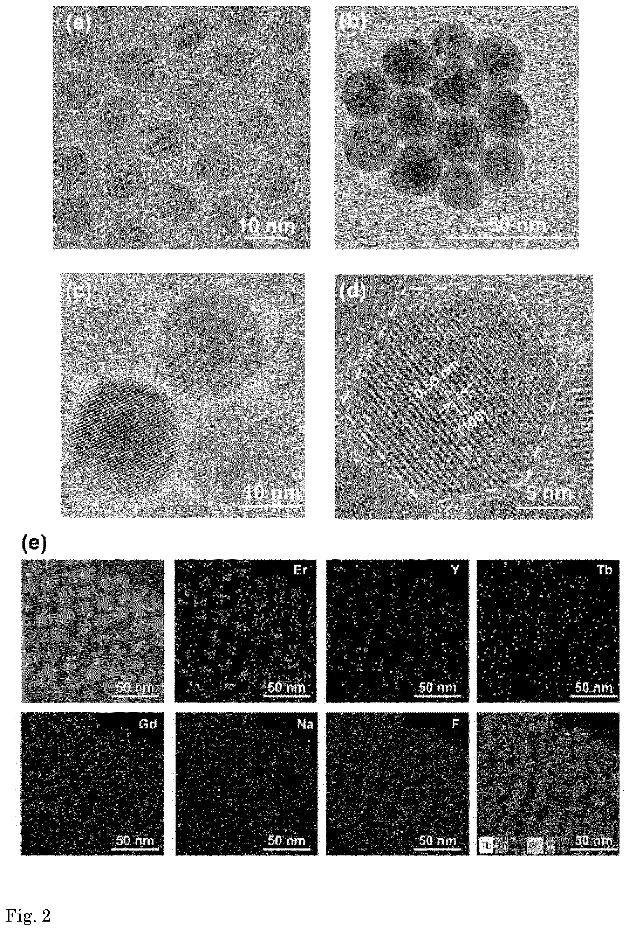

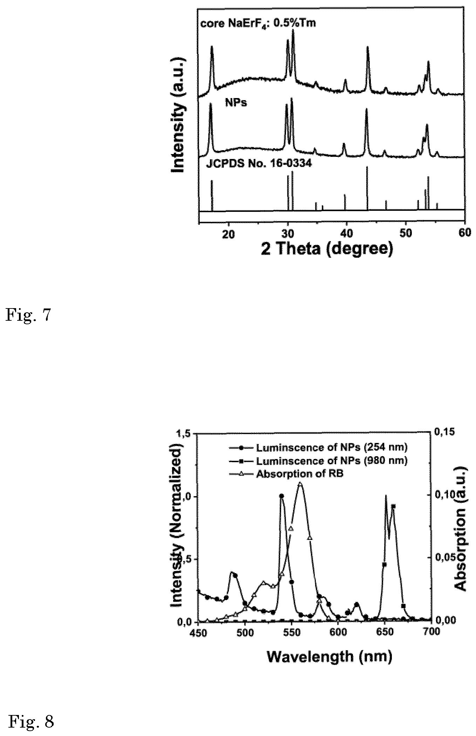

Transmission electron microscopy (TEM) images indicate that the core nanoparticles prepared in example 1 are spherical and monodispersed with the average diameter of ∼9 nm (figure 2a). The NaYF₄ buffer layer prevents energy transfer between the core and the outer layer. Figures 2b and 2c are TEM images of the nanoparticles, showing the uniform size and morphology of the nanoparticles with the average diameter of ca. 17 nm. Furthermore, the high-resolution TEM image (HRTEM) of an individual particle (figure 2d) exhibits clear lattice fringes of the hexagonal phase crystal (100) plane with the spacing of d(100) = 0.53 nm. The X-ray powder diffraction (XRD) pattern in figure 7 indicates that both the core nanoparticles and the multilayer nanoparticles exhibit the hexagonal phase. High-angle annular dark field scanning transmission electron microscopy (HAADF-STEM) images are shown in figure 2e, the heavier lanthanide elements (Er, Gd, and Tb) which appear brighter are distributed in the core and outer layer of the nanoparticles, whereas the lighter metal (Y) is distributed in the middle layer of the nanoparticles.

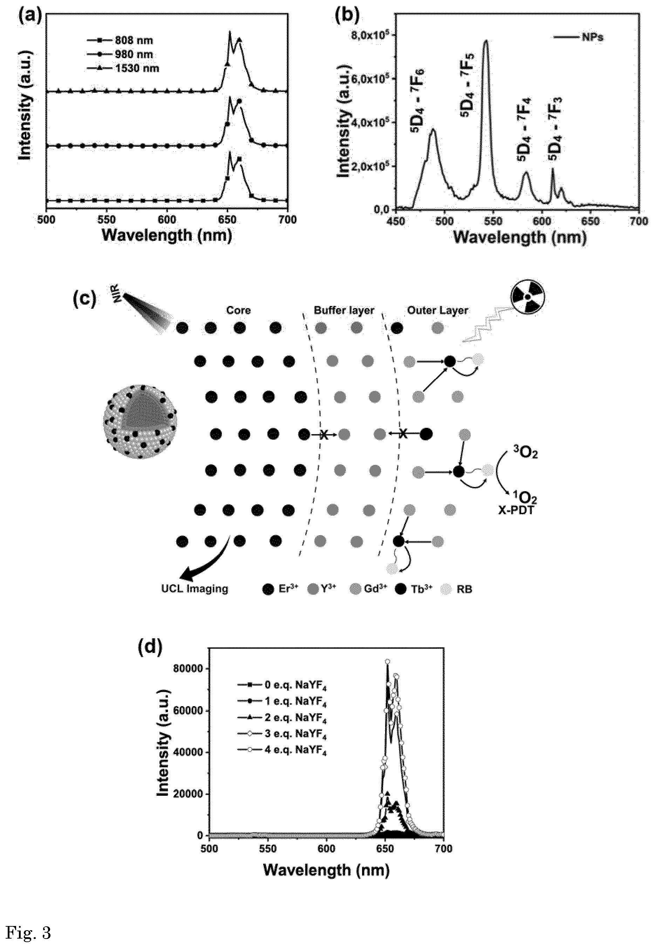

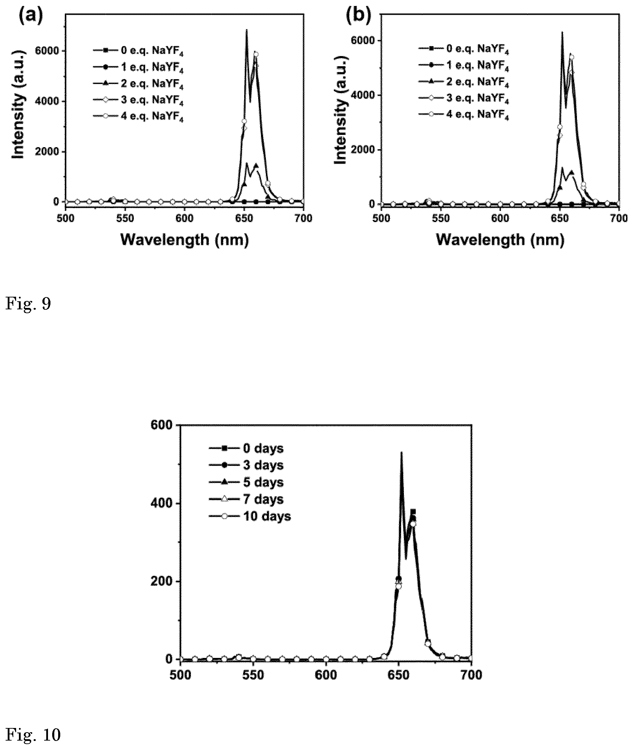

Figure 3a shows the upconversion luminescence spectra of the nanoparticles. Monochromic red emission around 650 nm originating from Tm³⁺ doped in the NaErF₄ core appears upon 808 nm, 980 nm or 1530 nm excitation. When excitation turns to X-ray the nanoparticles exhibit the characteristic emission of Tb³⁺ (figure 3b) which overlaps reasonably well with the rose bengal (RB) absorption (figure 8), indicating that excited energy of Tb³⁺ could be effectively transferred to the surface bounded RB through nonradiative resonant energy transfer and induces PDT (*i.e*., X-PDT). The NaYF₄ buffer layer plays a key role not only in minimizing the interaction between Er³⁺ ions in the core and the surface related quenching centers, *e.g.* defects, ligands, solvent molecules *etc*., but also in blocking the energy transfer pathway between the core and the X-ray active outer layer of the nanoparticle, thus enhancing the local density of excitation energy (*cf.* figure 3c). Regarding UCL imaging, the outer layer also plays as a layer that prevents interference by minimizing surface quenching. To optimize the thickness of the intermediate layer, the relationship between the UCL intensity of Er³⁺ and the distance between the core and surface of the nanoparticle, *i.e.* the buffer layer plus the outer layer, was measured using NIR excitation (see figures 3d and 9). The UCL intensity increases with the thickness of the middle layer until core/buffer layer/outer layer ratio reaches 1:3:3, corresponding to a 2.6 nm thick buffer layer and a 1.5 nm thick outer layer.

### Example 5 - Surface functionalization of Er@Y@Gd/Tb NPs

To functionalize the nanoparticles further for X-ray mediated PDT application, the as-synthesized oleic acid-capped nanoparticles (NPs) were coated with poly(allylamine) (PAAm) to form NPs-NH2 via ligand exchange. In detail, 4 mL as-synthesized Er@Y@Gd/Tb NPs (*x* = 3, referring to example 4) dispersed in cyclohexane and 4 mL 0.1 M HCl were mixed for 4 h. After washing with water twice, the nanoparticles were dispersed in 4 mL DMF and 200 µL PAAm (20 wt.%) aqueous solution was added in. The mixture was then stirred for 24 h. The nanoparticles were washed with water and DMF. Finally, the NPs-NH₂ were obtained and dispersed in DMF. To attach RB and FA covalently to NPs-NH2, 2 mg rose bengal hexanoic acid (RB-HA), 0.5 mg FA, 3 mg NHS and 3 mg EDC-HCl were mixed in 5 mL DMF at 25 °C for 2 h. Afterwards, 10 mg of NPs-NH2 was added and the reaction was run for 24 h. The obtained nanoparticles were then washed with DMF to remove the unreacted reagents. Finally, 10 mg PEG-NHS was reacted with the particles in 15 mL DMSO and 5 mL ethanol for 24h. After washing with water twice, the resulting nanoparticles, which will be referred to as upconversion luminescence-imaging-guided synergistic RT&X-PDT nanoplatforms (UIRPPs) were dispersed in phosphate buffered saline (PBS).

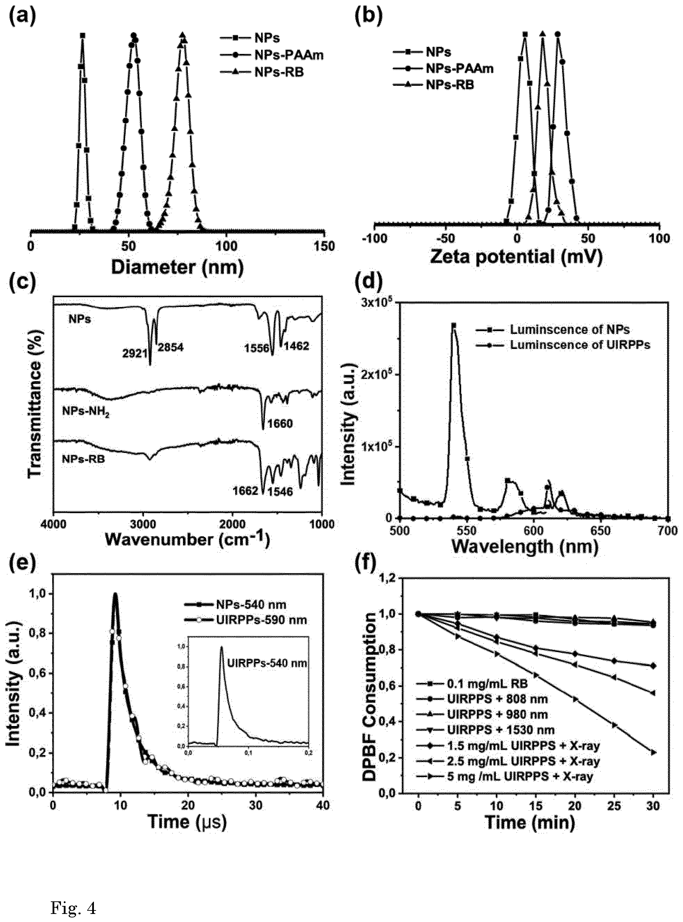

As shown in figure 4a and 4b, the nanoparticle diameter as obtained from dynamic light scattering and the zeta potential increased from ca. 26 nm to ca. 52 nm and from +5.5 mV to +30 mV, respectively, after amino group modification. These changes indicate that amine groups were successfully linked on the surface of the nanoparticles. Subsequently, RB and cancer targeting moiety-folic acid (FA) were covalently conjugated with NPs-NH₂ to form NPs-RB/FA, followed by surface modification with PEG-NHS to form UIRPPs and to improve further the biocompatibility and specificity. The diameter of UIRPPs increased to 77 nm and the zeta potential decreased to +17 mV. The successful preparation of UIRPPs was further supported by FTIR spectra (figure 4c). The oleic acid-capped NPs (NPs-OA) exhibited the asymmetric and symmetric stretching vibrations of methylene around 2921 and 2854 cm⁻¹, as well as the characteristic of carboxylic group around 1556 and 1462 cm⁻¹. PAAm coating revealed a N-H stretching vibration band at 1660 cm⁻¹, whereas the characteristic peaks of OA disappeared. Conjugating with the RB molecule brought in new bands at 1662 cm⁻¹ (C=O) and 1547 cm⁻¹ (N-H) of secondary amide. It deserves noting that these modifications had negligible influence on the upconversion luminescence (UCL) and its stability. Figure 10 is the ten days track of UCL spectrum of UIRPPs dispersed in water. Regarding UCL imaging, the neglected spectral overlap between the UCL of NPs and the RB absorption (*cf.* figure 8) indicates that UCL under NIR excitation can be used for imaging without inducing phototoxicity.

Regarding therapy, the multilayer UIRPP structure enables effective nonradiative energy transfer from donor atoms inside the outer layer of the nanoparticles to the surface-anchored photosensitizers. The X-ray active Tb³⁺ ions were doped solely in the outer layer and the thickness of the outer layer was controlled to be less than 2 nm, ensuring that the Tb³⁺ is distributed like ring rather than a like a ball. This structure facilitates energy transfer by optimizing the effective amount of energy donors and by ensuring a short distance to the anchored surface-anchored photosensitizers. Based on these multilayer, high surface-to-volume ratio nanoparticles, the loading efficiency was estimated high to be 12% (w/w) due to the covalent bonding. In the emission spectra of UIRPPs (*cf.* figure 4d), no Tb³⁺ luminescent peaks (in the blue and green range) were recorded due to the energy transfer from Tb³⁺ to RB and light absorption of RB. As a result, these multilayer UIRPPs show a FRET efficiency close to 100%, as can be seen in figure 4e. By fitting the data shown in figure 4e, a significant shortening of the decay of 540 nm emission from original 2.51 µs to 39 ns is observed, and a corresponding prolonging of RB excited state from pico- to nanoseconds to 2.54 µs In addition, one RB molecule has four iodine atoms, which could also contribute in absorbing X-rays for computed tomography (CT) imaging and enhancing the effects of radiotherapy.

### Example 6 - Singlet Oxygen Detection (¹O₂)

The efficacies of PDT activated with NIR laser or X-ray were estimated in solution by evaluating ¹O₂ generation efficiency using singlet oxygen detector diphenylisobenzofuran (DPBF). For the NIR triggered ¹O₂, 2 mL of UIRPPs-water solution and 10 µL (1 mg/mL) of DPBF-ethanol solution were mixed and kept in the dark overnight. The absorption intensity of DPBF at 410 nm was recorded every 5 min (5, 10, 15, 20, 25 and 30 min) after NIR laser irradiation (808 nm, 980 nm or 1530 nm) of 0.7 W/cm². For X-ray triggered ¹O₂, different concentration of UIRPPs and 10 µL (1 mg/mL) of DPBF-ethanol solution were mixed and kept in the dark overnight. The absorption intensity of DPBF at 410 nm was recorded every 5 min (at 5, 10, 15, 20, 25 and 30 min) after X-ray irradiation at 80 kV and 0.5 mA.

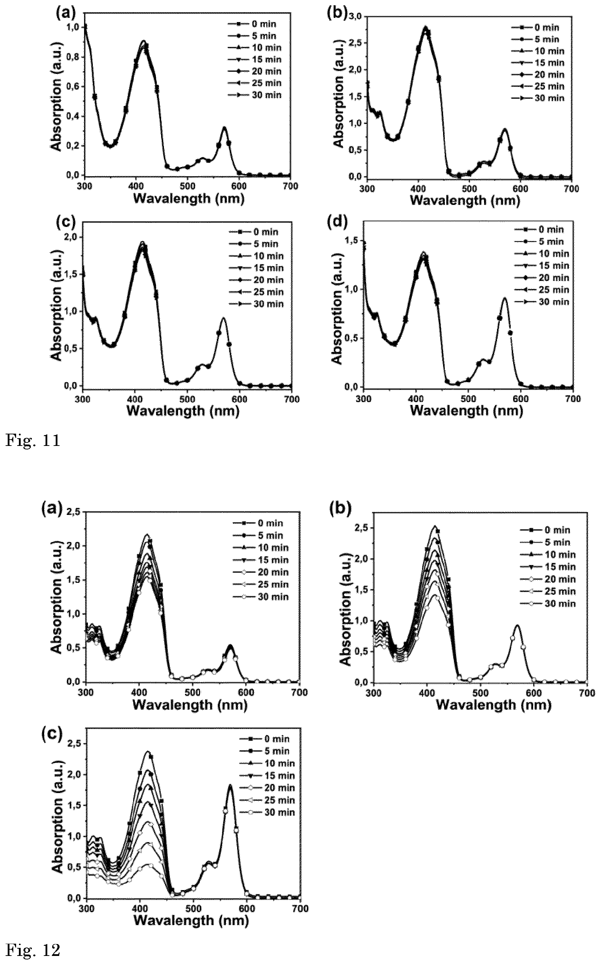

As shown in figures 4f, 11 and 12, the DPBF consumption of the 2 mg/mL UIRPPs aqueous solution remains almost constant with time upon NIR light irradiation (808 nm, 980 nm or 1530 nm) of 0.7 W/cm², indicating that NIR laser irradiation of UIRPPs generates negligible amounts of ¹O₂. DPBF absorption, however, does decline once UIRPPs in solution are irradiated with X-rays, even at very low concentration (1.5 mg/mL). With reference to the control groups (RB only) the exhibited linear relationship between ¹O₂ generation and concentration of UIRPPs and irradiation time, indicates a constant ¹O₂ generation rate. These results show the suitability of UIRPPs for imaging with minimal phototoxic effect as well as highly efficient X-PDT.

### Example 7 - UCL imaging and RT&X-PDT in vitro

To assess the application in cancer theranostics, UCL imaging and synergistic effect of RT&X-PDT of UIRPPs were tested *in vitro.* Breast carcinoma MCF-7 cells were maintained in the Dulbecco's modified Eagle medium (DMEM) under 5% CO₂ atmosphere at 37 °C. The cells were incubated in a special confocal well plate (1 × 10⁴ per well) for 24 h. Afterwards, the UIRPPs were added in the plate with 0 µg/mL, 50 µg/mL, 100 µg/mL, or 200 µg/mL, and incubation for 8 h. The nucleus was stained with 4',6-diamidino-2-phenylindole (DAPI), and the UCL imaging was measured by confocal microscopy with an irradiation wavelength of 980 nm and an emission wavelength of 650 nm or 540 nm. The UCL-RB was measured with 980 nm laser irradiation and 590 nm emission, the RB was measured with 540 nm irradiation and 590 nm emission.

To assess the toxicity of the UIRPPs and lasers, the MCF-7 cells were incubated in the 96-well plate (1 × 10⁴ per well) for 24 h. Then UIRPPs of different concentration groups (0, 0, 0, 0, 50, 100, 200, 400 µg/mL) were added in the plate, each group was with five parallels. The group with 0 µg/mL of UIRPPs were irradiated with 808 nm, 980 nm or 1530 nm laser for 15 min. Further incubated for 24 h, the cell viability was assessed using a standard MTS assay.

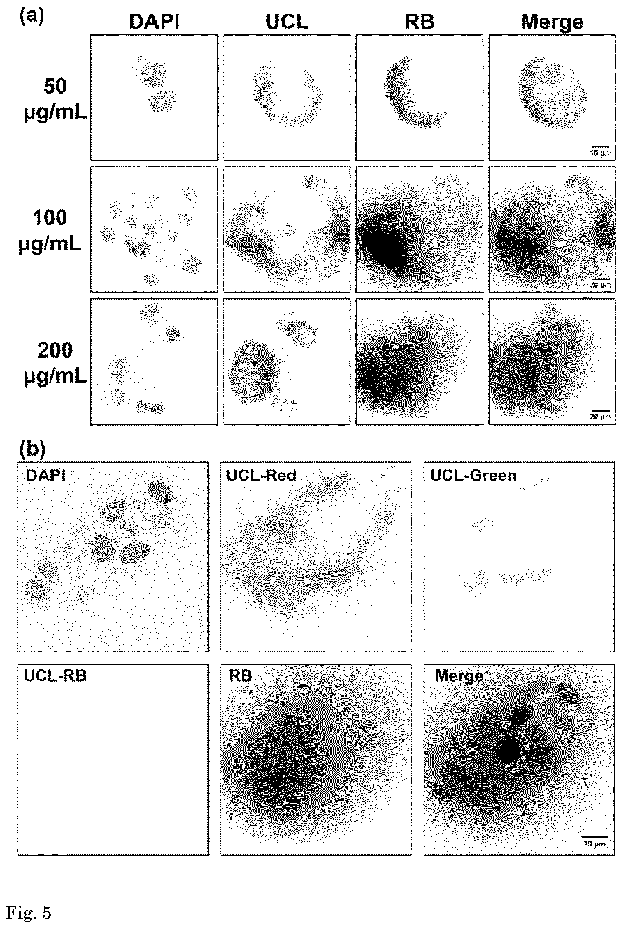

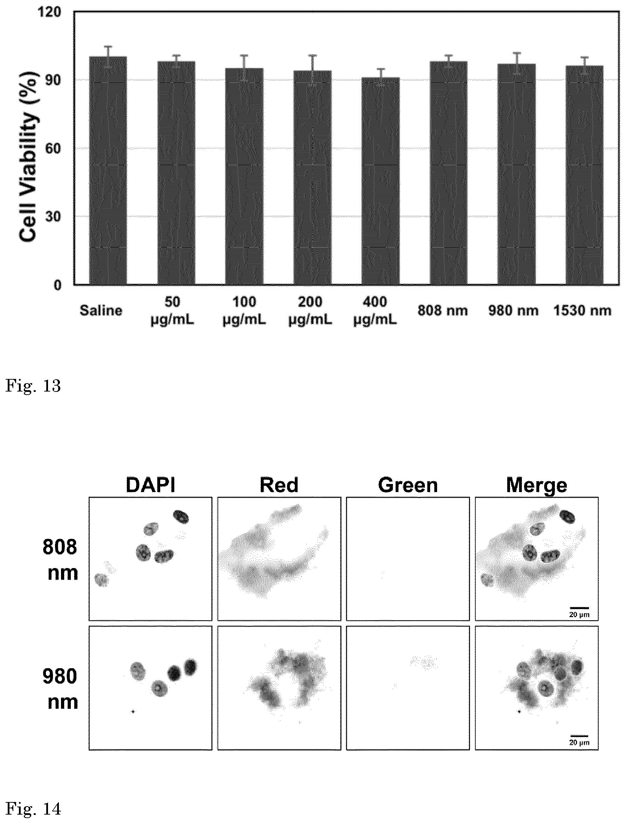

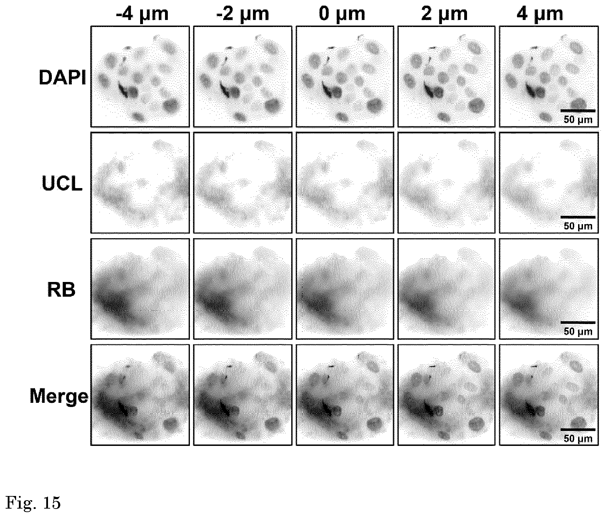

Figure 13 is the viability of a breast cancer line (MCF-7) subject to treatments. There was no obvious cytotoxicity of MCF-7 cells either incubated with UIRPPs, even at concentration as high as 400 µg/mL, or upon the NIR laser irradiation (808 nm, 980 nm or 1530 nm). As expected, 8 hours after MCF-7 cells incubated with UIRPPs (100 µg/mL) the cells emitted bright red UCL signals around the nuclei upon the laser irradiation at 980 or 808 nm (figure 14). It is worth noting that there was no green UCL, guaranteeing the desired monochromic red UCL for imaging. The nanoparticles were modified with folic acid (FA) to enhance the cellular uptake efficiency *via* FA-receptor-mediated endocytosis. The positive targeting was successful as is shown in figure 5a where the UCL signal increased sharply with the concentration of UIRPPs. The UCL imaging maintained high resolution with the concentration of UIRPPs varying from 50 to 200 µg/mL. To feature the advantage of imaging with UCL, a comparison was made with the fluorescence imaging using RB emission under 540 nm laser irradiation (*cf.* figure 5a). Although RB aided imaging increased also sharply with RB concentration, image became more and more blurry mainly due to scattering of the irradiation light. Figure 15 is the confocal images of Z-scan to exhibit the endocytosis and the fine resolution of UCL imaging. Photoactivity of RB subject to NIR laser irradiation was assessed (*cf.* figure 5b), neither UCL green nor RB signal was observed, supporting that bio-functionalized UIRPPs are suitable for the specific monochromic red UCL imaging without severe photodamage.

Regarding the PDT effect of the UIRPPs, the cells were separated in 8 groups (G1 to G8) in the 96-well plate, G1 and G5 were incubated with saline, G2, G3, G4 and G8 were incubated with UIRPPs of different concentrations (0, 50, 100, 200, 400 µg/mL), G6 were incubated with 12% RB (0, 6, 12, 24, 48 µg/mL) and G7 were incubated with Er@Y@Gd/Tb NPs. After 8h incubation all groups were washed with PBS. The saline (G1 and G5) were taken as the control groups, the G2, G3 and G4 were irradiated with the NIR laser (800 nm, 980 nm or 1530 nm) for 15 min at 0.7 W/cm². The G6, G7 and G8 were irradiated with X-rays for 45 min at 80 kV and 0.5 mA. After that the cells were cultured for another 48 h, the PDT efficacy of all groups was assessed by MTS assay.

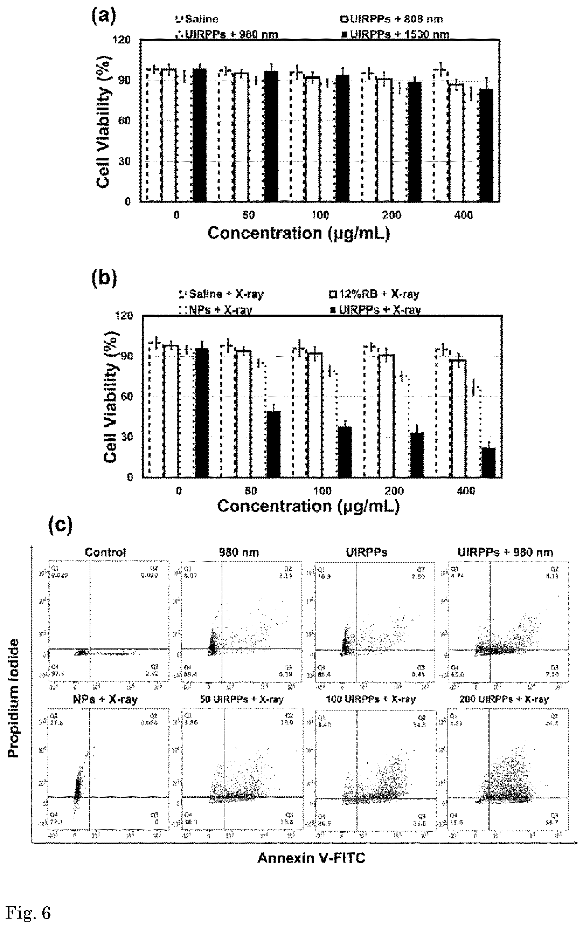

An MTS assay was used to evaluate the scatheless UCL imaging and the synergistic RT&X-PDT of UIRPPs. As shown in figure 6a, irradiation of NIR laser (808 nm, 980 nm or 1530 nm) did not induce statistical MCF-7 cell death, obviously there was not sufficient radical oxygen species (ROS) generation. In contrast, upon low dosage of X-ray irradiation (80 kV, 1.5 Gy), significant drops in cell viability were observed, as shown in figure 6b. For the UIRPPs + X-ray group, the number of the cell death increased with the concentration of UIRPPs and the synergistic RT&X-PDT led to ca. 80% of cell death when incubated with 400 µg/mL UIRPPs.

### Example 8 - Flow cytometry

Flow cytometry analysis was used to characterize the apoptosis of MCF-7 cells experiencing synergistic RT&X-PDT. For the flow cytometry analysis, the cells were trypsinized and stained by Annexin V-FITC and propidium iodide (Annexin V-FITC Apoptosis Staining/Detection Kit) to measure the cell apoptosis.

Annexin-V-fluorescein isothiocyanate (FITC) and propidium iodide (PI) staining were performed using an Annexin-V-FITC/PI kit (BD) according to the manufacturer's instructions. Briefly, MCF-7 cells were cultured in 6-well plates and then treated with different reagents (G1: saline, G2: saline, G3: 100 µg/mL UIRPPs, G4: 100 µg/mL G5: 100 µg/mL Er@Y@Gd/Tb NPs, G6: 50 µg/mL, UIRPPs, G7: 100 µg/mL UIRPPs, G8: 100 µg/mL UIRPPs) for 8 h, each with 3 parallels. The cells treated with saline (G1) were for the control. For the G2 and G4, the cells were exposed to the 980 nm laser for 15 min at 0.7 W/cm². And for the G5 to G8, the cells were irradiated with X-ray for 45 min at 80 kV and 0.5 mA. Then the cells were harvested using 0.05% trypsin after 24 h incubation and washed twice with cold PBS, and suspended in binding buffer. 1×10⁵ cells in 100 pL binding buffer were added to a tube and incubated with 5 pL of Annexin-V-FITC and 5 pL of propidium iodide (PI). Cells were gently mixed and incubated for 15 min at room temperature. 400 pL binding buffer was then added to each tube. The samples were analyzed by flow cytometry (BD Biosciences). Experiments were repeated three times.
As can be seen in figure 6c, the Annexin-V-FITC staining is for apoptotic cells and propidium iodide (PI) staining for the necrosis cells. The groups of MCF-7 cells incubated with saline, UIRPPs, simply upon irradiation of 980 nm laser, or incubated with UIRPPs plus 980 nm laser irradiation all remained very high survival rates. Upon X-ray irradiation, however, apoptosis of MCF-7 cells obviously occurred. In the "NPs + X-ray" group, the nanoparticles were not coupled with photosensitizers, and the X-ray radiation effects caused about 30% of cell deaths. In the "UIRPPs" group, there were only 38.3%, 26.5% or 15.6% alive cells after irradiation with X-ray corresponding to different concentrations of UIRPPs (50 µg/mL, 100 µg/mL or 200 µg/mL). The death ratio of cells was as high as 84.4%, fully showing the synergistic RT&X-PDT. These data show scatheless UCL imaging and synergistic highly efficient RT&X-PDT using nanoparticles according to an embodiments of the invention.

## Claims

1. Nanoparticle, comprising
- a core,
- an outer layer, and
- a buffer layer located between said core and said outer layer,
wherein the buffer layer prevents energy transfer between the core and the outer layer.

2. Nanoparticle according to claim 1, wherein the core, the outer layer, and the buffer layer are crystalline and have substantially the same crystal structure, preferably a hexagonal crystal structure.

3. Nanoparticle according to claim 1 or 2, wherein the core is an upconversion nanoparticle.

4. Nanoparticle according to any one of claims 1-3, wherein the core, the buffer layer, and/or the outer layer comprise an oxide or fluoride material comprising one or more rare-earth elements, preferably CaO-X₂O₃ wherein X is a rare-earth element, and/or NaXF₄, wherein X is a rare-earth element, optionally doped with one or more other rare-earth elements.

5. Nanoparticle according to any one of claims 1-4, further comprising one or more photosensitizers, radiosensitizers, and/or tumor targeting moieties attached to the outer layer.

6. Nanoparticle according to any one of claims 2-5, wherein the lattice mismatch between the buffer layer and the core and between the buffer layer and the outer layer is 25% or less, preferably 10% or less.

7. Nanoparticle according to any one of claims 1-6 for use in therapy.

8. Nanoparticle according to any one of claims 1-7 for use in treatment of a tumor.

9. Nanoparticle according to any one of claims 1-8 for use in *in vivo* imaging.

10. Method for the production of a nanoparticle according to claims 1-9, comprising the steps of
- heating of a solution comprising core precursors, followed by
- injection of a dispersion comprising buffer layer precursors, followed by
- injection of a dispersion comprising outer layer precursors.

11. Method according to claim 10, wherein the solution is heated to a temperature of 200 °C or higher, preferably 250 °C or higher, such as 300 °C or higher.

12. Method according to claim 10 or 11, wherein said solution and dispersions contain less than 200 ppm of water by total weight of the solution or dispersion, preferably less than 100 ppm, and wherein said solution and dispersions are more preferably substantially free of water.

13. Method according to any one of claims 10-12, further comprising the step of functionalization of the surface of the outer layer, wherein surface functionalization is preferably performed using one or more selected from the group consisting of poly(allylamine), poly(acrylamide) (PAAm), poly(acrylic acid) (PAA), and other PEG derivatives.

14. Method according to any one of claims 10-13, further comprising the step of covalently binding one or more photosensitizers and/or tumor targeting moieties to the nanoparticle or functionalized nanoparticle.

15. Method for *in vivo* imaging or treatment or both of a human or animal, comprising the steps of:
- administering nanoparticles according to any one of claims 1-9 to a patient,
- irradiating at least part of the patient's body with one or more types of radiation.

16. Use of a nanoparticle according to claims 1-9 for therapy, *in vivo* imaging, or both.
